(19) Europäisches Patentamt
European Patent Office
Office européen des brevets

(11) **EP 4 473 899 A1**

(12) **EUROPEAN PATENT APPLICATION**
published in accordance with Art. 153(4) EPC

(43) Date of publication:
**11.12.2024 Bulletin 2024/50**

(51) International Patent Classification (IPC):
*A61B 5/02* (2006.01)   *A61B 5/0245* (2006.01)
*A61B 5/11* (2006.01)   *A61B 5/113* (2006.01)

(21) Application number: 22924962.8

(22) Date of filing: 24.11.2022

(52) Cooperative Patent Classification (CPC):
A61B 5/02; A61B 5/0245; A61B 5/11; A61B 5/113

(86) International application number:
**PCT/JP2022/043269**

(87) International publication number:
**WO 2023/149057 (10.08.2023 Gazette 2023/32)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **01.02.2022 JP 2022014106**

(71) Applicant: **Heartlab, Inc.**
**Kobe-shi, Hyogo 650-0047 (JP)**

(72) Inventor: **ASANOI, Hidetsugu**
**Imizu-shi, Toyama 939-0364 (JP)**

(74) Representative: **Marks & Clerk LLP**
**15 Fetter Lane**
**London EC4A 1BW (GB)**

(54) **HEART SOUND EXTRACTION DEVICE, HEART SOUND EXTRACTION PROGRAM, RECORDING MEDIUM, AND MONITORING SYSTEM**

(57) A heart sound extraction device 3 comprises an acquisition unit 31 that, while a subject is lying on a sheet sensor that outputs a piezoelectric signal depending on the vibration applied, continuously acquires the piezoelectric signal output from the sheet sensor; a heart sound signal extraction unit 32c that extracts the heart sound signal of the subject from the piezoelectric signal; and a time window setting unit 32g that sets at least any of the following time windows in the extracted heart sound signal: (1) a time window in which the reciprocal of the standard deviation of respiratory frequency is maximum or equal to or greater than a predetermined value, (2) a time window in which the periodic respiratory power is maximum or equal to or greater than a predetermined value, (3) a time window in which the amplitude of the heart sound signal is maximum or equal to or greater than a predetermined value, (4) a time window in which the amplitude variation of the heart sound signal is minimum or equal to or less than a predetermined value, (5) a time window including the time zone of apnea in time window (2), (6) a time window in which the standard deviation of the heartbeat amplitude is minimum or equal to or less than a predetermined value, and (7) a time window in which the standard deviation of heartbeat intervals is minimum or equal to or less than a predetermined value.

Fig. 1

## Description

Technical Field

The present invention relates to technology for analyzing heart sound signals acquired without contacting or attaching a sensor directly to a subject.

Background Art

Heart sounds are made by the valves, myocardium, blood flow, etc. when the heart contracts and expands, and are classified into sounds I to IV.

Sound I occurs during ventricular contraction, and mainly includes mitral valve closure sound and aortic valve opening sound. Sound II occurs at the beginning of ventricular diastole, and mainly includes aortic valve closure sound and pulmonary valve closure sound. Sound III occurs after sound II, i.e., during the rapid filling period of the ventricles in early diastole, and is caused by the sudden blockage of blood flow from the atria to the ventricles by the ventricular wall. The more sudden the blockage, the louder the sound, and the sound is likely to occur due to a decrease in ventricular compliance during diastole. Sound IV is caused by the sudden blockage of blood flow ejected into the ventricle by atrial contraction by the ventricular wall during the rise in ventricular end-diastolic pressure just before sound I.

In a healthy adult, generally only sounds I and II can be heard, and sounds III and IV are not confirmed. That is, sounds III and IV are treated as extra heart sounds. If these sounds are confirmed, there may be some kind of disease in the heart.

For example, sound III is heard in mitral regurgitation, aortic regurgitation, ventricular septal defect, myocardial infarction, ischemic heart disease, cardiomyopathy, myocarditis, and the like, and is particularly so important in heart failure that it is used as a sign of congestion to determine hospital admission and discharge. Sound IV is heard in pulmonary hypertension, aortic stenosis, ischemic heart disease, myocarditis, cardiomyopathy, and the like (NPL 1).

Citation List

Non-patent Literature

NPL 1: Takashi MURO, "Auscultation - Sounds III and IV - Tracking auscultatory findings to understand pathological changes," online, May 2013, Medical Tribune, internet, URL: https://midori-hp.or. jp/wp/wp-content/uploads/2015/08/Medical-Tribune201305.pdf

Summary of Invention

Technical Problem

Since sounds III and IV differ in the mechanism of occurrence from sounds I and II, it takes skill to correctly determine the presence or absence of these sounds even when listening with a stethoscope. However, extra heart sounds are very important findings because they occur or increase when the diseases described in paragraph [0005] are developed or exacerbated. However, the only way to determine the presence or absence of extra heart sounds is for a doctor with advanced auscultation skills to perform auscultation, or for the patient himself/herself to place an electronic stethoscope or a high-performance microphone on his/her chest to collect phonocardiogram. The heart sounds of outpatients (patients at home) could not be monitored daily for use in early detection of the onset or exacerbation of disease.

In recent years, the introduction of systems that allow medical institutions to use ICT to monitor patients receiving home care or home nursing has been attracting attention. For example, the present applicant is developing a system in which a sheet-like body motion sensor is laid on the bed of a home care patient with heart failure, the patient's respiratory signal is extracted from the output signals (body motion signals) of the body motion sensor, and the severity of heart failure is predicted from the reciprocal of the standard deviation of respiratory frequency (RST).

Further, in such a monitoring system, if the presence or absence of extra heart sounds can be identified from noisy output signals from the body motion sensor etc., the patient himself/herself will not need to place a sensor, such as an electronic stethoscope, on his/her chest. Accordingly, daily monitoring will be easier, and the onset and exacerbation of diseases related to extra heart sounds, such as heart failure, will be detectable. Therefore, this allows for early detection and early treatment.

However, the heart sound signal included in the output signals from the body motion sensor is much weaker than the signals of body motion, respiratory effort (breathing), and pulse (heart rate), and is therefore easily affected by noise etc. It was thus difficult to extract data (time windows) showing heart sound signals (waveforms) that could be used to correctly determine the presence or absence of extra heart sounds. Therefore, currently, the only way to determine extra heart sound has been to listen to the heartbeat with less noise collected with an electronic stethoscope or a high-performance microphone, or to look at the heartbeat waveform, which placed a heavy burden on patients and medical staff.

The present invention was made to solve the above problems, and an object thereof is to extract data suitable for analysis from heart sound signals extracted from a noisy sensor, such as a body motion sensor.

Solution to Problem

In order to achieve the above object, the present invention includes the following aspects.

Item 1.

A heart sound extraction device comprising:

an acquisition unit that, while a subject is lying on a sheet sensor that outputs a piezoelectric signal depending on the vibration applied, continuously acquires the piezoelectric signal output from the sheet sensor;
a heart sound signal extraction unit that extracts the heart sound signal of the subject from the piezoelectric signal; and
a time window setting unit that sets at least any of the following time windows in the extracted heart sound signal:

(1) a time window in which the reciprocal of the standard deviation of respiratory frequency is maximum or equal to or greater than a predetermined value,
(2) a time window in which the periodic respiratory power is maximum or equal to or greater than a predetermined value,
(3) a time window in which the amplitude of the heart sound signal is maximum or equal to or greater than a predetermined value,
(4) a time window in which the amplitude variation of the heart sound signal is minimum or equal to or less than a predetermined value,
(5) a time window including the time zone of apnea in time window (2),
(6) a time window in which the standard deviation of the heartbeat amplitude is minimum or equal to or less than a predetermined value, and
(7) a time window in which the standard deviation of heartbeat intervals is minimum or equal to or less than a predetermined value.

Item 2.

The heart sound extraction device according to Item 1, wherein the time window setting unit sets a signal of a time window in which the reciprocal of the standard deviation of respiratory frequency is maximum from the piezoelectric signal.

Item 3.

The heart sound extraction device according to Item 1, wherein the time window setting unit sets a time window including the time zone of apnea in a time window in which the periodic respiratory power is maximum from the piezoelectric signal.

Item 4.

The heart sound extraction device according to Item 1, wherein the time window setting unit sets a time window in which the standard deviation of the heartbeat amplitude is minimum from the piezoelectric signal.

Item 5.

The heart sound extraction device according to any one of Items 1 to 4, further comprising a display unit that displays a waveform within the set time window of the extracted heart sound signal.

Item 6.

A heart sound extraction program for making a computer work as each unit of the heart sound extraction device according to any one of Items 1 to 5.

Item 7.

A computer-readable recoding medium that records the heart sound extraction program according to Item 6.

Item 8.

A monitoring system that monitors the state of a subject based on the heart sound of the subject, comprising:

a sheet sensor that outputs a piezoelectric signal depending on the vibration applied; and
a heart sound extraction device that extracts a heart sound signal from the piezoelectric signal output from the sheet sensor while the subject is lying on the sheet sensor,
the heart sound extraction device being the heart sound extraction device according to any one of Items 1 to 5.

Advantageous Effects of Invention

According to the present invention, it is possible to extract data suitable for analysis from heart sound signals extracted from a noisy sensor, such as a body motion sensor.

Brief Description of Drawings

Fig. 1 is a block diagram showing the schematic structure of a monitoring system according to an embodiment of the present invention.
Fig. 2 schematically shows an example of installation of a measuring device.
Fig. 3 shows an example of a piezoelectric signal acquired in the subject's lying period.
Fig. 4 is a detailed functional block diagram of a heart sound extraction unit.
Fig. 5 (a) shows spectral distribution by frequency analysis in a time window with maximum RST, and (b) shows the waveforms of the respiratory signal, CSR signal, and noise component in this time window.
Fig. 6 (a) shows spectral distribution by frequency analysis in a time window with maximum CSR, and (b) shows the waveforms of the respiratory signal, CSR signal, and noise component in this time window.
Fig. 7 is a detailed functional block diagram of a heart sound analysis unit.
Fig. 8 shows an example of the waveform of heart sound signal extracted by the heart sound extraction unit.
Fig. 9 shows an example of the amplitude variation waveform of the heart sound signal.
Fig. 10 shows an example of distribution information of peak time intervals detected from the amplitude variation waveform.
Fig. 11 shows an example of distribution information in a histogram format.
Fig. 12 is a graph plotting peaks of the amplitude variation waveform of the heart sound signal including sound III, with the time on the x-axis and the time interval with the immediately preceding peak on the y-axis.
Fig. 13 is a graph plotting peaks of the amplitude variation waveform of the heart sound signal not including sounds III and IV, with the time on the x-axis and the time interval with the immediately preceding peak on the y-axis.
Fig. 14 is a graph plotting peaks of the amplitude variation waveform of the heart sound signal including sound IV, with the time on the x-axis and the time interval with the immediately preceding peak on the y-axis.
Fig. 15 shows an example of a graph for determining extra heart sounds.
Fig. 16 shows the waveforms of heart sound signal etc. extracted from a time window with maximum RST.
Fig. 17 shows the amplitude variation waveform of the heart sound signal shown in Fig. 16.
Fig. 18 shows distribution information of peak time intervals detected from the amplitude variation waveform shown in Fig. 17.
Fig. 19 shows the distribution information shown in Fig. 18 in a histogram format.
Fig. 20 shows the waveforms of heart sound signal etc. extracted from the time window of apnea in a time window with maximum CSR.
Fig. 21 shows the histogram of the distribution of peak time intervals detected from the amplitude variation waveform of the heart sound signal shown in Fig. 20.

Fig. 22 shows the waveforms of heart sound signal etc. extracted from a time window in which the SD of the heartbeat amplitude is minimum.

Fig. 23 shows the amplitude variation waveform of the heart sound signal shown in Fig. 22.

Fig. 24 shows distribution information of peak time intervals detected from the amplitude variation waveform shown in Fig. 23.

Fig. 25 shows the distribution information shown in Fig. 24 in a histogram format.

Fig. 26 shows the waveforms of heart sound signal etc. extracted from a time window in which the SD of RR intervals is minimum.

Fig. 27 shows the amplitude variation waveform of the heart sound signal shown in Fig. 26.

Fig. 28 shows distribution information of peak time intervals detected from the amplitude variation waveform shown in Fig. 27.

Fig. 29 shows the distribution information shown in Fig. 28 in a histogram format.

Fig. 30 shows the waveforms of heart sound signal etc. extracted from the time window of apnea in a time window with maximum CSR.

Fig. 31 shows the histogram of the distribution of peak time intervals detected from the amplitude variation waveform of the heart sound signal shown in Fig. 30.

Fig. 32 shows the waveforms of heart sound signal etc. extracted from a time window in which the SD of the heartbeat amplitude is minimum.

Fig. 33 shows the histogram of the distribution of peak time intervals detected from the amplitude variation waveform of the heart sound signal shown in Fig. 32.

Description of Embodiments

An embodiment of the present invention is described below with reference to the attached drawings. The present invention is not limited to the following embodiment.

System Structure

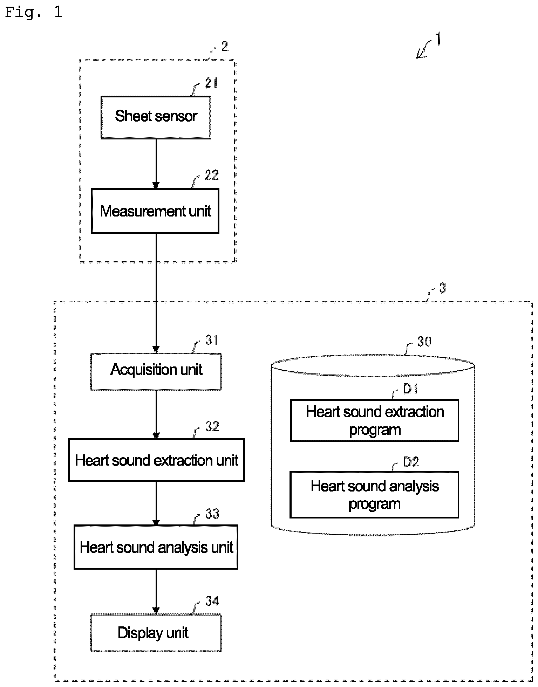

Fig. 1 is a block diagram showing the schematic structure of a monitoring system 1 according to an embodiment of the present invention. The monitoring system 1 monitors the state of a subject based on the heart sounds of the subject, and comprises a measuring device 2 and a management device 3. The subject targeted in the present embodiment is mainly a patient with heart disease staying in a place other than a medical institution, such as home; however, the place where the subject stays is not particularly limited. In addition, in the present embodiment, the disease of the subject is a disease that can be diagnosed or predicted from the heart sounds; however, it is not necessary to particularly limit the disease, and the subject may be a healthy person.

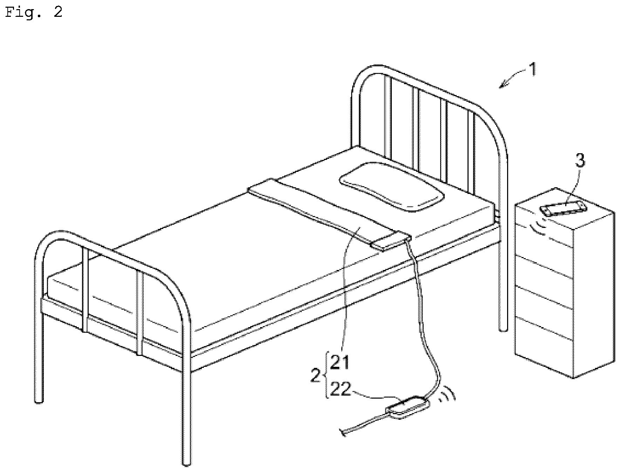

Fig. 2 schematically shows an example of installation of the measuring device 2. The measuring device 2 is provided in a place where the subject lives (e.g., subject's home or elderly care facility), and comprises a sheet sensor 21 and a measurement unit 22.

As shown in Fig. 2, the sheet sensor 21 comprises a piezoelectric element, and is provided on the bed the subject uses on a daily basis. In Fig. 2, the bed sheet and comforter laid on the sheet sensor 21 are not shown. While the subject is lying, the sheet sensor 21 outputs a piezoelectric signal depending on the vibration applied from the subject's body.

In the present embodiment, "lying" means that the subject is lying regardless of whether the subject is asleep or not. Therefore, the sheet sensor 21 may be provided not only in a sleeping area such as bed, but also an area where the patient can lie down and rest (sofa etc.).

The measurement unit 22 is connected to the sheet sensor 21, and AD-converts the piezoelectric signal generated by the sheet sensor 21. Further, the measurement unit 22 has the function of communicating with a smartphone 4 using Bluetooth (registered trademark), and the AD-converted piezoelectric signal is temporarily saved in the smartphone 4, and then transmitted to the management device 3.

The management device 3 corresponds to the heart sound extraction device and heart sound analysis device recited in the claims, and is provided at a medical institution or in the cloud. The management device 3 can be composed of a general-purpose computer, and comprises, as hardware structures, a processor such as a CPU or GPU, a main storage device such as DRAM or SRAM (not shown), and an auxiliary storage device 30 such as an HDD or SSD. The auxiliary storage device 30 stores various programs for operating the management device 3, such as a heart sound extraction program D1 and a heart sound analysis program D2. The heart sound extraction program D1 and the heart sound analysis program D2 may be downloaded to the management device 3 via a communication network, such as the Internet, or the heart sound extraction program D1 and the heart sound analysis program D2 may be recorded on a computer-readable non-transitory recording medium, such as an SD card, and then installed into the management device 3 via the storage medium.

The management device 3 comprises an acquisition unit 31, a heart sound extraction unit 32, a heart sound analysis unit 33, and a display unit 34 as functional blocks. The acquisition unit 31, the heart sound extraction unit 32, and the heart sound analysis unit 33 each may be realized in hardware using a logic circuit etc., or may be realized in software using a CPU etc. When the acquisition unit 31 and the heart sound extraction unit 32 are realized in software, it can be realized in such a manner that the CPU of the management device 3 reads the heart sound extraction program D1 to the main storage device and executes it. When the heart sound analysis unit 33 is realized in software, it can be realized in such a manner that the CPU of the management device 3 reads the heart sound analysis program D2 to the main storage device and executes it.

The management device 3 may be composed of multiple devices. For example, the parts that function as the acquisition unit 31 and the heart sound extraction unit 32 may be configured as a heart sound extraction device, and the parts that function as the heart sound analysis unit 33 and the display unit 34 may be configured as a heart sound analysis device.

The acquisition unit 31 is a functional block that acquires the piezoelectric signal from the sheet sensor 21. The acquisition unit 31 does not always need to obtain the piezoelectric signal, and may obtain the piezoelectric signal at least while the subject is lying. If the sampling frequency of the piezoelectric signal is high, the acquisition unit 31 may perform downsampling to reduce the amount of data.

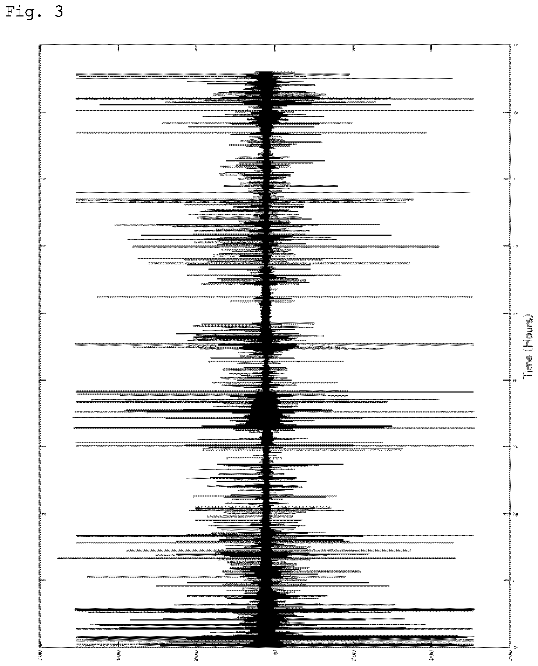

Fig. 3 is an example of the piezoelectric signal acquired in the subject's lying period and downsampled to 400 Hz. In most of the lying period, the piezoelectric signal includes body motion, breathing, heartbeat, and many other noise components.

Overview of Heart Sound Extraction Method

The heart sound extraction unit 32 shown in Fig. 1 is a functional block that extracts the heart sound signal of the subject from the piezoelectric signal. The heart sound signal in the piezoelectric signal is much weaker than the signals of body motion, breathing, and heart rate, and is therefore easily affected by noise etc. It was thus difficult with conventional technology to extract heart sound signals (waveforms) that could be used to correctly determine the presence or absence of extra heart sounds. Accordingly, the heart sound extraction unit 32 automatically sets a time window that can withstand high-precision heart sound analysis for the heart sound signal extracted from the piezoelectric signal.

In the present embodiment, the time window is at least one of the following time windows:

(1) a time window in which the reciprocal of the standard deviation of respiratory frequency (RST) is maximum
(2) a time window in which the periodic respiratory power (CSR) is maximum
(3) a time window in which the amplitude of the extracted heart sound signal is maximum
(4) a time window in which the amplitude variation of the extracted heart sound signal is minimum
(5) a time window of apnea in time window (2) above
(6) a time window in which the standard deviation (SD) of the heartbeat amplitude is minimum
(7) a time window in which the SD of heartbeat intervals (RR intervals) is minimum

The present inventor focused on the fact that there are relatively few components other than the heart sound signals within these time frames, and found that the use of the heart sound signals within these time windows allows for high-precision heart sound analysis.

The reason for selecting time window (1) is that breathing is periodic when RST is large, making it easier to remove the influence of the respiratory signal. The reason for selecting time window (2) is that since the respiratory arrest time is long when CSR is large, it is less affected by breathing. The reason for selecting time window (3) is that a strong heart sound signal is present in this time window. The reason for selecting time window (4) is that a stable heart sound signal is present in this time window. The reason for selecting time window (5) is that during apnea, the influence of breathing can be completely removed. The reason for selecting time window (6) is that when the amplitude of the heartbeat is large, the volume (amplitude) of the heart sound may also fluctuate. The reason for selecting time window (7) is to remove the influence of variations in heart sound intervals due to variations in RR intervals. The following explains a specific example of setting a time window for extra heart sound determination.

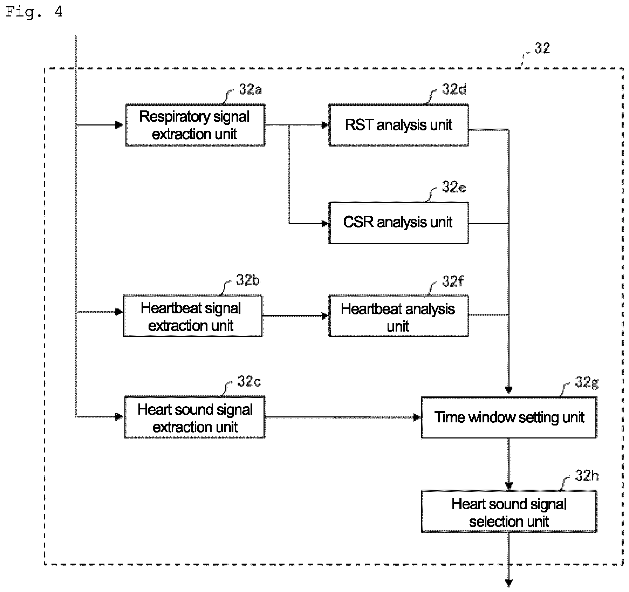

Fig. 4 is a detailed functional block diagram of the heart sound extraction unit 32. The heart sound extraction unit 32 comprises a respiratory signal extraction unit 32a, a heartbeat signal extraction unit 32b, a heart sound signal extraction unit 32c, an RST analysis unit 32d, a CSR analysis unit 32e, a heartbeat analysis unit 32f, a time window setting unit 32g, and a heart sound signal selection unit 32h. The piezoelectric signal output from the acquisition unit 31 is input to the respiratory signal extraction unit 32a, heartbeat signal extraction unit 32b, and heart sound signal extraction unit 32c.

The respiratory signal extraction unit 32a extracts the respiratory signal from the piezoelectric signal. Since the respiratory signal has a relatively high signal level, it is easier to extract it while removing noise than the heart sound signal, and analysis throughout the recording time is possible. In the present embodiment, the respiratory signal extraction unit 32a removes noise from the piezoelectric signal at 4000 Hz, then finally downsamples the signal to 4 Hz, and extracts the respiratory signal through a band-pass filter of 0.008 to 0.6 Hz. This is output to the RST analysis unit 32d and CSR analysis unit 32e.

The RST analysis unit 32d calculates RST from the respiratory signal. In the present embodiment, the RST analysis unit 32d moves a 5-minute time window of the respiratory signal by 50 seconds to extract a signal within the window, and performs frequency analysis on the signal of each time window using the maximum entropy method. The CSR analysis unit 32e extracts the envelope waveform (CSR waveform) of the respiratory signal, and performs frequency analysis on the envelope waveform for each time window using the maximum entropy method. RST and CSR grades are calculated from the results of the two frequency analyses.

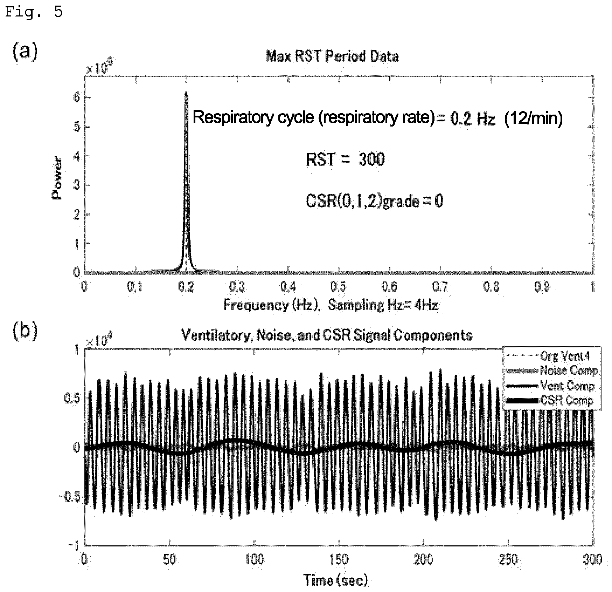

Fig. 5 (a) shows spectrum distribution obtained by frequency analysis in a time window with maximum RST, and Fig. 5 (b) shows the waveforms of the respiratory signal (black line), CSR signal (red line), and noise component (blue line) in this time window. It can be seen that when RST is maximum, breathing is highly periodic.

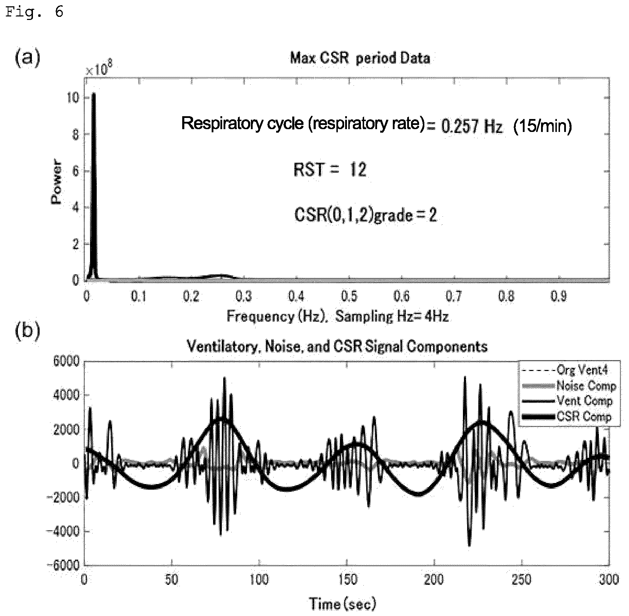

Fig. 6 (a) shows spectrum distribution obtained by frequency analysis in a time window with maximum CSR, and Fig. 6 (b) shows the waveforms of the respiratory signal (black line), CSR signal (red line), and noise component (blue line) in this time window. From these waveforms, it can be seen that while the CSR signal is significantly below zero, breathing stops, and the noise component is extremely small.

The heartbeat signal extraction unit 32b extracts a pulsation signal (heartbeat signal) from the piezoelectric signal. In the present embodiment, the heartbeat signal extraction unit 32b removes noise from the piezoelectric signal at 4000 Hz, then finally downsamples the signal to 30 Hz, and extracts the heartbeat signal through a band-pass filter of 0.8 to 2 Hz.

The heartbeat analysis unit 32f moves a 5-minute time window by 50 seconds for the heartbeat signal all night to extract a signal in the window, and calculates the SD of the heartbeat amplitude and the SD of RR intervals in each time window.

RST of each time window calculated by the RST analysis unit 32d, CSR of each time window calculated by the CSR analysis unit 32e, and the SD of the heartbeat amplitude and the SD of RR intervals in each time window calculated by the heartbeat analysis unit 32f are output to the time window setting unit 32g.

The heart sound signal extraction unit 32c extracts a heart sound signal from the piezoelectric signal. In the present embodiment, the heart sound signal extraction unit 32c downsamples the piezoelectric signal from 4000 Hz to 400 Hz, removes noise, and extracts the heart sound signal through a band-pass filter of 25 to 100 Hz.

Setting of Time Window Suitable for Heart Sound Analysis

The time window setting unit 32g sets a time window suitable for heart sound analysis in the heart sound signal based on information from the RST analysis unit 32d, CSR analysis unit 32e, heartbeat analysis unit 32f, and heart sound signal extraction unit 32c. Specifically, the time window setting unit 32g sets a signal of at least one of the following time windows:

(1) a time window in which RST is maximum
(2) a time window in which CSR is maximum
(3) a time window in which the amplitude of the extracted heart sound signal is maximum
(4) a time window in which the amplitude variation of the extracted heart sound signal is minimum
(5) a time window of apnea in time window (2) above
(6) a time window in which the SD of the heartbeat amplitude is minimum
(7) a time window in which the SD of RR intervals is minimum The number of time windows set by the time window setting unit 32g may be single or multiple; however, among these time windows, time windows (1), (2), and (5) are preferred. Further, time windows (2) and (5) are particularly preferred because they are not affected by respiratory signals.

Time window (1) is not limited to the time window with maximum RST, but may include a time window with RST equal to or greater than a predetermined value. Time window (2) is also not limited to the time window with maximum CSR, but may include a time window with CSR equal to or greater than a predetermined value. Time window (3) is also not limited to the time window in which the amplitude of the heart sound signal is maximum, but may include a time window in which the amplitude of the heart sound signal is equal to or greater than a predetermined value. Time window (4) is not limited to the time window in which the amplitude variation of the heart sound signal is minimum, but may include a time window in which the amplitude variation of the heart sound signal is equal to or less than a predetermined value. Time window (6) is also not limited to the time window in which the SD of the heartbeat amplitude is minimum, but may include a time window with in which the SD of the heartbeat amplitude is equal to or less than a predetermined value. Time window (7) is also not limited to the time window in which the SD of RR intervals is minimum, but may include a time window in which the SD of RR intervals is equal to or less than a predetermined value.

The length of each time window is 5 minutes in the present embodiment as described above, but may be appropriately changed.

The heart sound signal selection unit 32h selects a heart sound signal within the set time window from the heart sound signal extracted by the heart sound signal extraction unit 32c. In the present embodiment, the heart sound signal selection unit 32h cuts the heart sound signal within the time window from the heart sound signal, outputs it to the heart sound analysis unit 33, and displays the waveform of the heart sound signal within the time window on the display unit 34. The medical staff observe the waveform of the heart sound signal (more desirably together with the waveform of the heartbeat signal), thereby determining the presence or absence of extra heart sounds.

The heart sound signal selection unit 32h may display a frame showing a time window on the waveform of the heart sound signal without cutting out the heart sound signal within the time window, or may display the waveform within the time window in a manner distinguishable from other waveforms. As a result, the medical staff can identify the portion of the heart sound signal suitable for determining the presence or absence of extra heart sounds. Alternatively, the heart sound signal selection unit 32h may be configured only to cut out the heart sound signal within the time window and to output it to the heart sound analysis unit 33, and not to display the waveform.

Analysis of Heart Sound

The heart sound analysis unit 33 shown in Fig. 1 is a functional block that analyzes the heart sound signal to thereby support the prediction of the subject's state. As explained in the Background Art section, if extra heart sounds are confirmed in the heart sound signal, there may be some kind of disease in the heart. Therefore, the heart sound analysis unit 33 particularly analyzes the presence or absence of extra heart sounds to support the prediction of the subject's state.

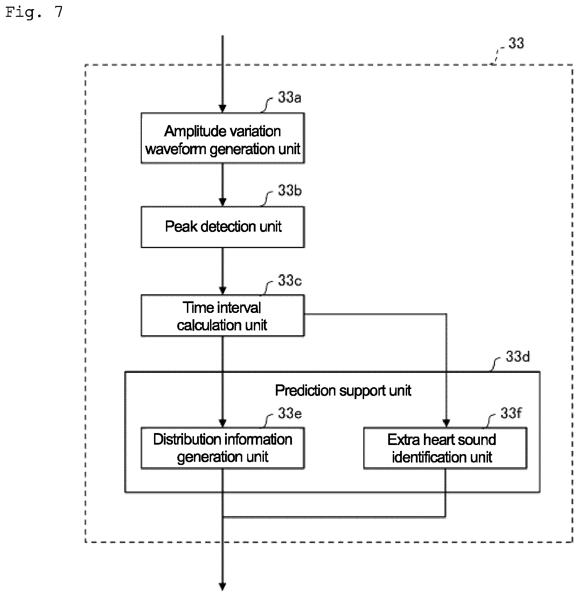

Fig. 7 is a detailed functional block diagram of the heart sound analysis unit 33. The heart sound analysis unit 33 comprises an amplitude variation waveform generation unit 33a, a peak detection unit 33b, a time interval calculation unit 33c, and a prediction support unit 33d.

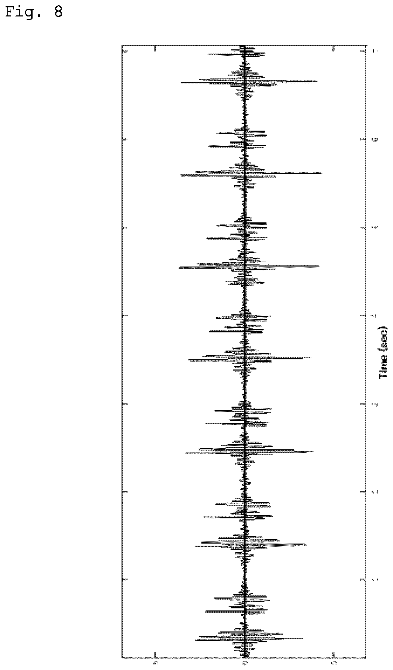

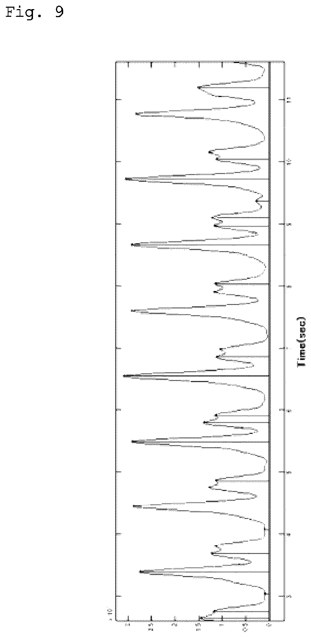

The amplitude variation waveform generation unit 33a is a functional block that generates the amplitude variation waveform of the heart sound signal. This function is explained based on Figs. 8 and 9.

Determination of Presence or Absence of Extra Heart Sounds

Fig. 8 is an example of the waveform of the heart sound signal extracted by the heart sound extraction unit 32. In Fig. 8, waveforms with large and small amplitudes appear to repeat regularly; however, actually, the amplitude size may be random due to the influence of denoising process etc.

The amplitude variation waveform generation unit 33a sets an envelope tangent to each wave of the heart sound signal in each of the positive and negative regions for the heart sound signal of Fig. 8, and corrects the negative envelope of the two envelopes to 0, thereby generating the amplitude variation waveform shown in Fig. 9.

The peak detection unit 33b shown in Fig. 7 is a functional block that detects peaks in the amplitude variation waveform. In the present embodiment, peaks refer to maximum points in the amplitude variation waveform. Fig. 9 shows peaks with dots.

The time interval calculation unit 33c shown in Fig. 7 is a functional block that calculates the time interval of each peak. In Fig. 9, the interval of perpendicular lines extending from points indicating the peaks to the time axis corresponds to the time interval of each peak. In the following explanation, the time interval may be simply referred to as the "interval."

The prediction support unit 33d shown in Fig. 7 is a functional block that supports the prediction of the subject's state based on the time intervals. In the present embodiment, the prediction support unit 33d supports the prediction of the subject's state by generating information suggesting the presence or absence of extra heart sounds in the heart sound signal, and/or automatically determining the presence or absence of extra heart sounds. In order to realize this function, the prediction support unit 33d comprises a distribution information generation unit 33e and an extra heart sound identification unit 33f.

As the information suggesting the presence or absence of extra heart sounds, the distribution information generation unit 33e generates distribution information indicating the distribution of the time intervals calculated by the time interval calculation unit 33c. The generated distribution information is displayed on the display unit 34.

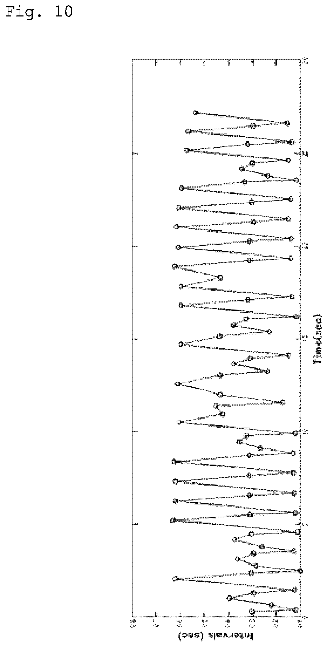

Fig. 10 shows an example of the distribution information. In this figure, time intervals between each peak and its immediately preceding peak are indicated with circles. From this figure, it can be intuitively understood that the time intervals include three groups: a group of about 0.1 seconds, a group of about 0.3 to 0.5 seconds, and a group of about 0.6 seconds.

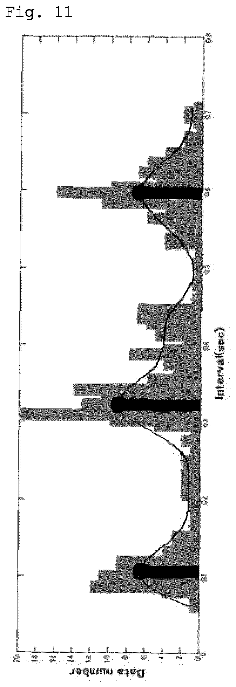

The distribution information generation unit 33e can also generate the distribution information in a histogram format. Fig. 11 shows an example of the distribution information in a histogram format. In this figure, the horizontal axis represents the time interval with the immediately preceding peak, and the vertical axis represents the number of peaks that have this time interval. From this histogram, it can be intuitively understood that the peak time intervals can be divided into 3 groups.

The number of time interval groups serves as an indicator to predict the presence or absence of extra heart sounds. In general, the heart sound of a healthy adult includes only sounds I and II in one heartbeat period (RR interval). In that case, there is the following tendency: interval between sound II and sound I in the next heartbeat period < interval between sound I and sound II. Therefore, the number of peak time interval groups is 2.

On the other hand, when sound III is present, there is the following tendency: interval between sound II and sound III < interval between sound I and sound II < interval between sound III and sound I in the next heartbeat period. Further, when sound IV is present in place of sound III (sounds III and IV are rarely present at the same), there is the following tendency: interval between sound IV and sound I in the next heartbeat period < interval between sound I and sound II < interval between sound II and sound IV. Therefore, when sound III or IV is present, the number of peak time interval groups is 3.

Thus, from the distribution information generated by the distribution information generation unit 33e, if the number of peak time interval groups can be understood, it is possible to predict the presence or absence of extra heart sounds in the heart sound without referring to the heartbeat signal. That is, in the heart sound signal shown in Fig. 8, the number of peak time interval groups in the amplitude variation waveform is 3, whereby it can be predicted that extra heart sounds are present.

When peaks are accurately detected by the peak detection unit 33b, the average time interval is calculated for each of the above groups, and the average total value is approximately equal to the average heart sound cycle. Accordingly, when the heartbeat is measured, the accuracy of the detection of peaks by the peak detection unit 33b can be determined by comparing the above total value with the average RR interval of the heartbeat. That is, the closer the above total value is to the average RR interval, the more accurately peaks are detected based on the waveform of the heart sound, which indicates a high reliability of the prediction of extra heart sounds.

Therefore, when multiple time windows are selected, it is preferable to use a prediction result with high reliability among prediction results obtained from heart sound signals extracted from the respective time windows.

Determination of Type of Extra Heart Sound

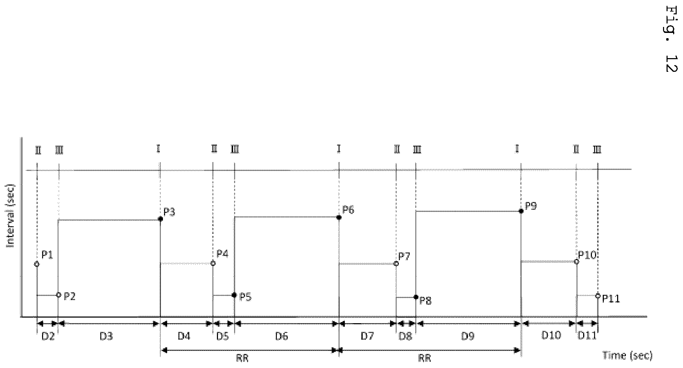

Since the interval between sound II and sound III is almost equal to the interval between sound IV and sound I in the next heartbeat period, the presence or absence of extra heart sounds can be predicted from the distribution information; however, it is difficult to determine whether the type of extra heart sound is sound III or sound IV. In contrast, the extra heart sound identification unit 33f shown in Fig. 7 has the function of automatically determining not only the presence or absence of extra heart sounds in the heartbeat signal, but also the type of extra heart sound, based on the peak time intervals. The determination method by the extra heart sound identification unit 33f is explained below with reference to Figs. 12 to 14.

Fig. 12 is a graph plotting peaks of the amplitude variation waveform of the heart sound signal including sound III, with the time on the x-axis and the time interval with the immediately preceding peak on the y-axis. This graph shows 11 peaks P1 to P11. The height of each peak is equal to the time interval with the immediately preceding peak. When the time interval between (n-1)th peak Pn-1 and nth peak Pn is taken as Dn, the height of peak Pn is Dn. That is, all the quadrilaterals in the graph are squares. Each peak is associated with symbol I, II, or III to indicate the type of heart sound; however, these are shown for convenience only. The extra heart sound identification unit 33f does not determine into which heart sound each peak is classified.

The extra heart sound identification unit 33f searches for maximum and minimum peaks from the peaks. The maximum peak refers to a peak higher than the preceding and following peaks, and the minimum peak refers to a peak lower than the preceding and following peaks. That is, the maximum peak satisfies the following condition:

$$\text{A: } Dn{-}1 < Dn > Dn{+}1,$$

and the minimum peak satisfies the following condition:

$$\text{B: } Dn{-}1 > Dn < Dn{+}1.$$

For example, peak P3 is a maximum peak because it satisfies D2<D3>D4 and satisfies condition A. Peaks P6 and P9 are also maximum peaks because they satisfy condition A.

Peak P5 is a minimum peak because it satisfies D4>D5<D6 and satisfies condition B. Peak P8 is also a minimum peak because it satisfies condition A.

On the other hand, peak P4 does not correspond to either a maximum peak or a minimum peak because it satisfies D3>D4>D5 and does not satisfy condition A or B. Similarly, peaks P7 and P10 do not correspond to either a maximum peak or a minimum peak.

In Fig. 12, the maximum and minimum peaks are indicated with black circles, and peaks that do not correspond to either a maximum peak of a minimum peak are indicated with white circles. For peaks P1, P2, and P11, time intervals necessary to determine whether they are maximum or minimum peaks within the graph range are omitted; thus, they are not subjects to this determination and are indicated with white circles.

Subsequently, the extra heart sound identification unit 33f selects any maximum peak as a first peak from the searched maximum peaks, selects the minimum peak immediately following the first peak as a second peak, and selects the maximum peak immediately following the second peak as a third peak. In this case, the time interval between the first and third peaks can be regarded as equal to the RR interval (hereinafter, RR) of the heartbeat. In Fig. 12, the first peak, second peak, and third peak correspond to a combination of peaks P3, P5, and P6, and a combination of peaks P6, P8, and P9.

Subsequently, the extra heart sound identification unit 33f calculates the ratio R of the time interval between the second and third peaks to the time interval between the first and second peaks. For example, when the first peak, second peak, and third peak are peaks P3, P5, and P6, respectively, the following equation holds:

$$R = (D4+D5)/D6 \qquad (C)$$

As described later, the ratio R serves as an indicator for determining the presence or absence of extra heart sounds in the heart sound signal and the type of extra heart sound.

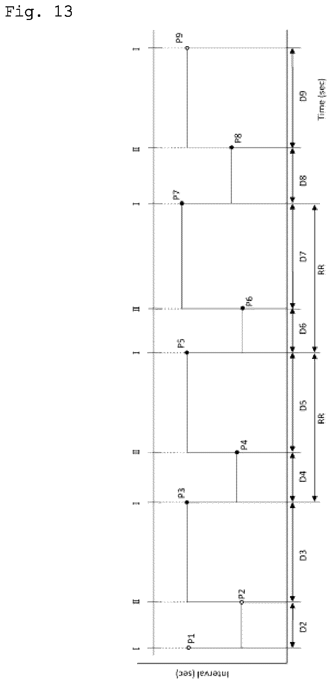

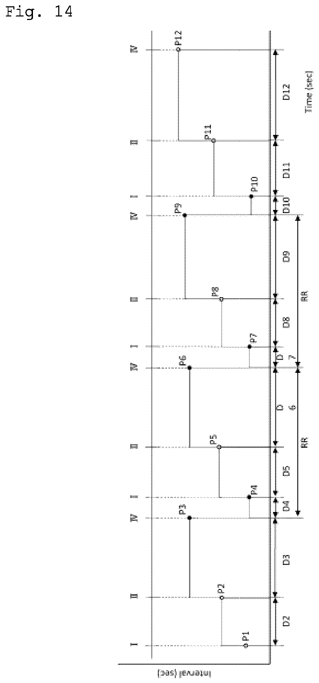

Fig. 13 is a graph plotting peaks of the amplitude variation waveform of the heart sound signal not including sounds III and IV, with the time on the x-axis and the time interval with the immediately preceding peak on the y-axis, and Fig. 14 is a graph plotting peaks of the amplitude variation waveform of the heart sound signal including sound IV, with the time on the x-axis and the time interval with the immediately preceding peak on the y-axis. The definition of each symbol in these graphs is the same as that of Fig. 12. The calculation of the ratio R from the graphs of Figs. 13 and 14 is explained below.

The graph shown in Fig. 13 shows 9 peaks P1 to P9. Each peak is associated with symbol I or II; however, these are shown for convenience only. The extra heart sound identification unit 33f does not determine into which heart sound each peak is classified.

The extra heart sound identification unit 33f searches for maximum peaks that satisfy condition A and minimum peaks that satisfy condition B from the peaks. The maximum peaks correspond to peaks P3, P5, and P7, and the minimum peaks correspond to peaks P4, P6, and P8. Peaks P1, P2, and P9 are indicated with white circles. Since time intervals necessary to determine whether they are maximum or minimum peaks within the graph range are omitted, these white circles mean that they are not subjects to this determination. In Fig. 14, there are no peaks that do not correspond to the maximum or minimum peaks.

Subsequently, the extra heart sound identification unit 33f selects a first peak, a second peak, and a third peak from the searched maximum and minimum peaks, and calculates the ratio R of the time interval between the second and third peaks to the time interval between the first and second peaks. For example, when the first peak, second peak, and third peak are peaks P3, P4, and P5, respectively, the following equation holds:

$$R = D4/D5 \qquad (D).$$

The graph shown in Fig. 14 shows 12 peaks P1 to P12. Each peak is associated with symbol I, II, or IV; however, these are shown for convenience only. The extra heart sound identification unit 33f does not determine into which heart sound each peak is classified.

The extra heart sound identification unit 33f searches for maximum peaks that satisfy condition A and minimum peaks that satisfy condition B from the peaks. The maximum peaks correspond to peaks P3, P6, and P9, and the minimum peaks correspond to peaks P4, P7, and P10.

Subsequently, the extra heart sound identification unit 33f selects a first peak, a second peak, and a third peak from the searched maximum and minimum peaks, and calculates the ratio R of the time interval between the second and third peaks to the time interval between the first and second peaks. For example, when the first peak, second peak, and third peak are peaks P3, P4, and P6, respectively, the following equation holds:

$$R = D4/(D5+D6) \qquad (E)$$

If the heart sound signal includes sound III, as shown in Fig. 12, there is the following tendency: interval between sound II and sound III < interval between sound I and sound II < interval between sound III and sound I in the next heartbeat period. Therefore, the maximum peak corresponds to sound I, and the minimum peak corresponds to sound III. That is, the ratio R calculated by formula (C) means interval between sound I and sound III/interval between sound III and sound I in the next heartbeat period.

On the other hand, if the heart sound signal includes only sounds I and II, as shown in Fig. 13, there is the following tendency: interval between sound II and sound I in the next heartbeat period < interval between sound I and sound II. Therefore, the maximum peak corresponds to sound I, and the minimum peak corresponds to sound III, as in Fig. 12. That is, the ratio R calculated by formula (D) means interval between sound I and sound II/interval between sound II and sound I in the next heartbeat period. Since sound III is emitted after sound II, the ratio R calculated by formula (C) tends to be larger than the ratio R calculated by formula (D).

Further, if the heart sound signal includes sound IV, as shown in Fig. 14, there is the following tendency: interval between sound IV and sound I in the next heartbeat period < interval between sound I and sound II < interval between sound II and sound IV. Therefore, the maximum peak corresponds to sound IV, and the minimum peak corresponds to sound I. That is, the ratio R calculated by formula (E) means interval between sound IV and sound I in the next heartbeat period/interval between sound I and sound IV. Since the interval between sound IV and sound I in the next heartbeat period is very short, the ratio R calculated by formula (E) tends to be smaller than the ratios R calculated by formulas (C) and (D).

Therefore, by setting 5 thresholds for the ratio R, it is possible to determine the presence or absence of extra heart sounds in the heart sound signal, and whether the type of extra heart sound is sound III or sound IV. In the present embodiment, thresholds a, b, c, d, and e (a<b<c<d<e) are set, and if it is clear that there are three peak time interval groups in paragraph [0055], the extra heart sound identification unit 33f can determine that sound IV is likely to be present when R is smaller than threshold c, and that sound III is likely to be present when R is larger than threshold c. Further, when a<R<b, the probability of the presence of sound IV is increased, and when d≤R<e, the probability of the presence of sound III is increased. The values of a, b, c, d, and e can be appropriately set depending on, for example, the sex and heart rate (RR interval) of the subject.

For example, for both a male and a female, a and b can be set as follows:

$$a = \frac{0.1}{(60 \div \text{heart rate} - 0.1)}$$

$$b = \frac{0.2}{(60 \div \text{heart rate} - 0.2)}$$

c is an average ratio when there is no sound III or IV, and can be set as follows for a male:

$$c = \frac{(-0.00158 \times \text{heart rate} + 0.392)}{[60 \div \text{heart rate} - (-0.00158 \times \text{heart rate} + 0.392)]}$$

For a female, c can be set as follows:

$$c = \frac{(-0.00158 \times \text{heart rate} + 0.412)}{[60 \div \text{heart rate} - (-0.00158 \times \text{heart rate} + 0.412)]}$$

Further, d and e can be set as follows for a male:

$$d = \frac{(-0.00158 \times \text{heart rate} + 0.492)}{[60 \div \text{heart rate} - (-0.00158 \times \text{heart rate} + 0.492)]}$$

$$e = \frac{(-0.00158 \times \text{heart rate} + 0.592)}{[60 \div \text{heart rate} - (-0.00158 \times \text{heart rate} + 0.592)]}$$

For a female, d and e can be set as follows:

$$d = \frac{(-0.00158 \times \text{heart rate} + 0.512)}{[60 \div \text{heart rate} - (-0.00158 \times \text{heart rate} + 0.512)]}$$

$$e = \frac{(-0.00158 \times \text{heart rate} + 0.612)}{[60 \div \text{heart rate} - (-0.00158 \times \text{heart rate} + 0.612)]}$$

The determination results are displayed on the display unit 34.

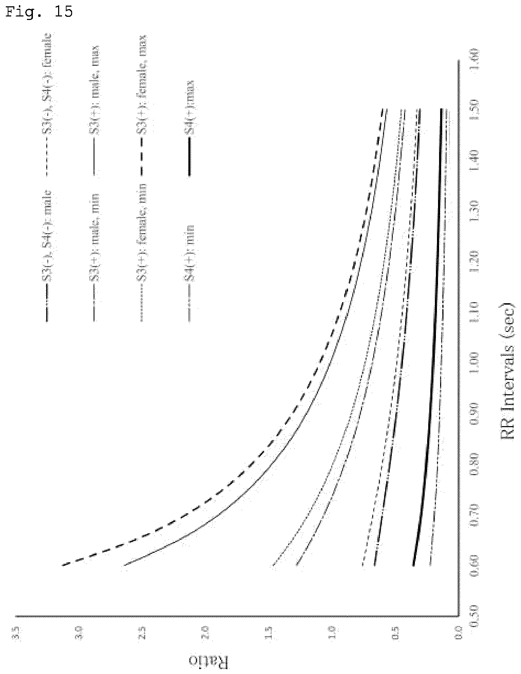

Fig. 15 shows an example of a graph for determining extra heart sounds based on the ratio R. In this graph, when the ratio R according to RR intervals is plotted, if the male is located in the area above the "S3(+): male, min" curve and the female is located in the area below the "S3(+): female, min" curve, it is determined that sound III is present. Further, if both are located in the area below the "S4(+): max" curve, it is determined that sound IV is present.

As described above, the extra heart sound identification unit 33f searches for maximum and minimum peaks from the amplitude variation waveform of the heart sound signal, selects first, second, and third peaks from the searched maximum and minimum peaks, calculates the ratio R of the time interval between the second and third peaks to the time interval between the first and second peaks, and determines the presence or absence of extra heart sounds in the heart sound signal and the type of extra heart sound based on the ratio R. The extra heart sound identification unit 33f may select multiple combinations of first, second, third peaks. In this case, the above determination may be made based on the average ratio R calculated from each combination.

Brief Summary

In the present embodiment, a signal of a specific time window is selected from the piezoelectric signal from the sheet sensor while the subject is lying, and components other than the heart sound are removed from the selected signal. Accordingly, the heart sound signal of the subject can be extracted with high accuracy using the sheet sensor. This makes it possible to acquire the heart sound signal of a subject staying at home using the sheet sensor, and to automatically determine the presence or absence of extra heart sounds etc. by analyzing the heart sound signal. Therefore, the state of a subject under home care can be monitored remotely based on the heart sound of the subject, and the burden on the subject and medical staff can be significantly reduced.

Further, in the present embodiment, peak time intervals in the amplitude variation waveform of the heart sound signal acquired from the subject are calculated, and distribution information indicating the distribution of the time intervals is generated. This makes it possible to predict the presence or absence of extra heart sounds, which takes skill to listen to, even without referring to the heartbeat signal. Further, maximum and minimum peaks that satisfy specific conditions are searched from the peaks, and from the ratio of time intervals in the consecutive maximum peak, minimum peak, and maximum peak (first to third peaks), it is possible to determine not only the presence or absence of extra heart sounds, but also the type of extra heart sound. This can support the prediction of the subject's state.

Additional Notes

The embodiment of the present invention is described above; however, the present invention is not limited to the above embodiment, and various modifications can be made without departing from the gist thereof.

In the above embodiment, the heart sound signal of the subject is acquired by extraction from the piezoelectric signal of the sheet sensor; however, the present invention is not limited thereto. For example, the heart sound signal of the subject may be acquired by using an electronic stethoscope or a high-performance microphone, and the signal may be analyzed to determine the presence or absence of extra heart sounds.

Examples

Examples of the present invention are described below; however, the present invention is not limited to the following Examples. In each Example, the measuring device 2 of the above embodiment was used to extract the heart sound signal of the subject from the piezoelectric signal of the sheet sensor, and the heart sound signal was analyzed in the management device 3.

Example 1

In Example 1, the target was subject X, who had been diagnosed for the presence of sound III in heart sounds by routine examination. Heart sound signals were extracted from the signals of the following time windows (1) and (5) to (7) in the piezoelectric signal obtained while subject X was lying on the sheet sensor, and extra heart sounds were determined based on peak time intervals detected from the amplitude variation waveform of the heart sound signals.

(1) a time window with maximum RST
(5) a time window of apnea in time window (2) above
(6) a time window in which the SD of the heartbeat amplitude is minimum
(7) a time window in which the SD of RR intervals is minimum.

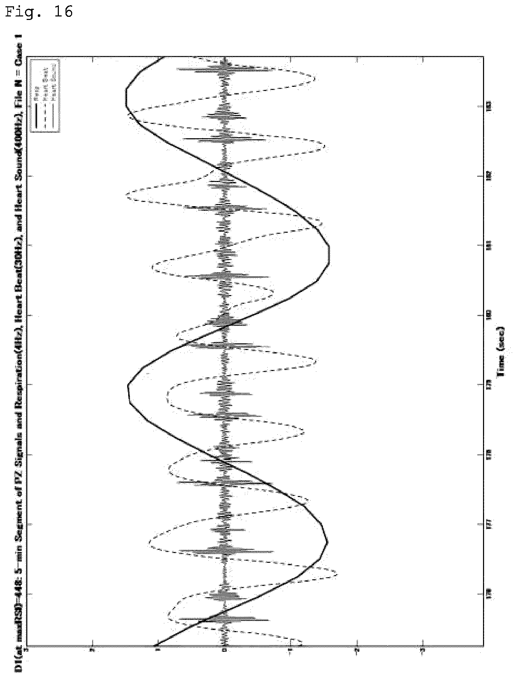

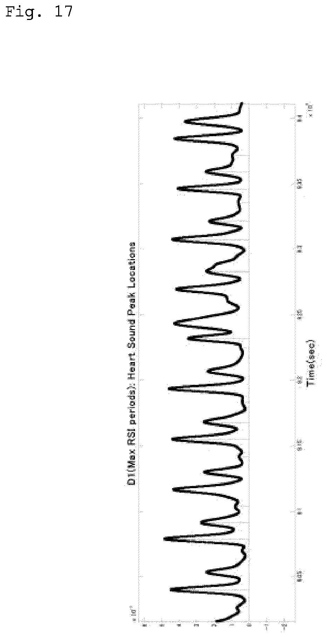

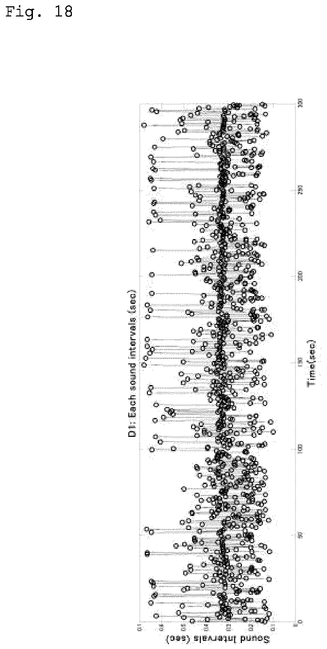

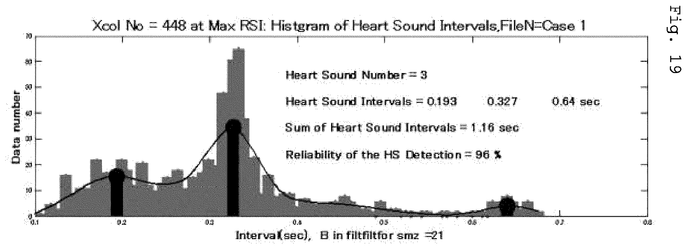

Fig. 16 shows the waveforms of the heart sound signal (black), heartbeat signal (red), and respiratory signal (blue) extracted from time window (1). Fig. 17 shows the amplitude variation waveform of the heart sound signal shown in Fig. 16. Fig. 18 shows distribution information indicating the distribution of peak time intervals detected from the amplitude variation waveform shown in Fig. 17, and Fig. 19 shows the distribution information in a histogram format.
Since the peak time intervals are roughly divided into 3 groups in Figs. 17 to 19, it can be predicted that the heart sound signals include extra heart sounds. The average time interval of each group was 0.193 sec, 0.327 sec, and 0.64 sec, and their total value (predicted heartbeat cycle) was 1.16 sec, whereas the average RR interval was 1.112 sec. Therefore, the prediction reliability was 1.112/1.16 = 96%.

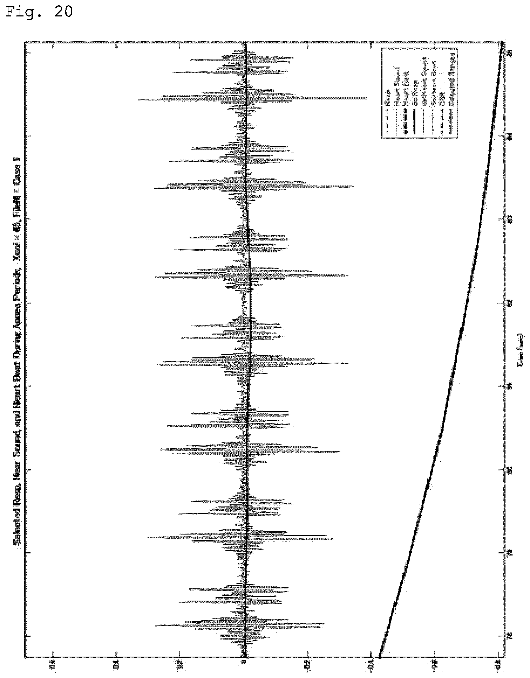

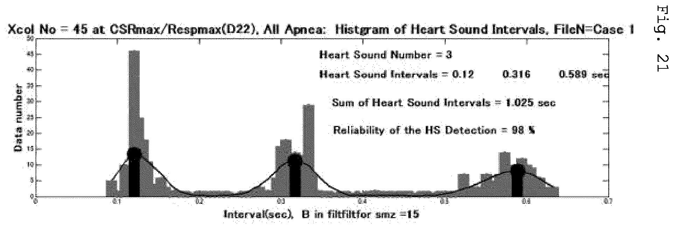

Fig. 20 shows the waveforms of the heart sound signal (black), CSR signal (red), and respiratory signal (blue) extracted from time window (5). Fig. 21 shows the histogram of the distribution of peak time intervals detected from the amplitude variation waveform of the heart sound signal shown in Fig. 20.
Since the peak time intervals are roughly divided into 3 groups in Fig. 21, it can be predicted that the heart sound signals include extra heart sounds. The average time interval of each group was 0.12 sec, 0.316 sec, and 0.589 sec, and their total value (predicted heartbeat cycle) was 1.025 sec, whereas the average RR interval was 1.009 sec. Therefore, the prediction reliability was 1.025/1.009 = 98%.

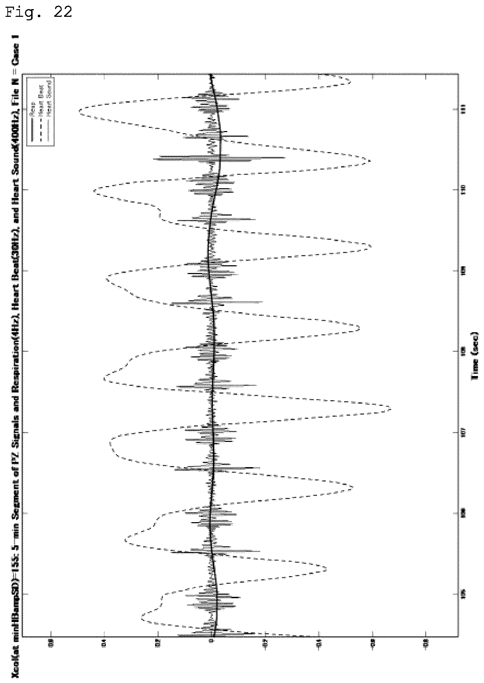

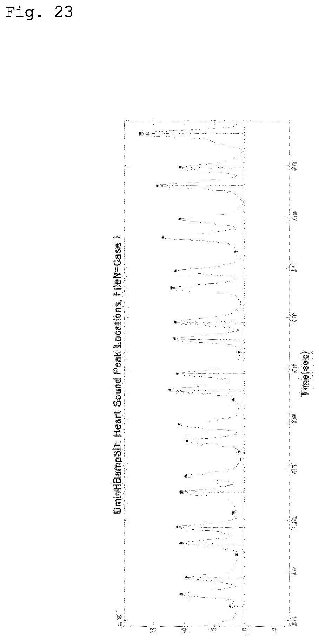

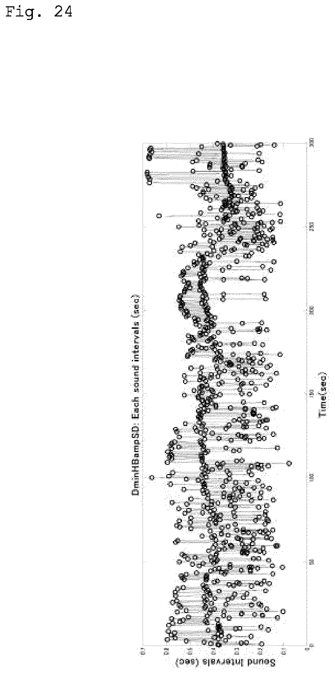

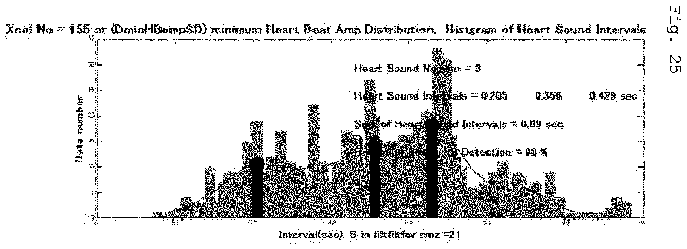

Fig. 22 shows the waveforms of the heart sound signal (black), heartbeat signal (red), and respiratory signal (blue) extracted from time window (6). Fig. 23 shows the amplitude variation waveform of the heart sound signal shown in Fig. 22. Fig. 24 shows distribution information indicating the distribution of peak time intervals detected from the amplitude variation waveform shown in Fig. 23, and Fig. 25 shows the distribution information in a histogram format.
In Figs. 24 and 25, groups of peak time intervals are not clear; however, as a result of automatically analyzing the histogram, it was determined that they were divided into 3 groups. It can be predicted that the heart sound signals include extra heart sounds. The average time interval of each group was 0.205 sec, 0.356 sec, and 0.429 sec, and their total value (predicted heartbeat cycle) was 0.99 sec, whereas the average RR interval was 0.973 sec. Therefore, the prediction reliability was 0.973/0.99 = 98%.

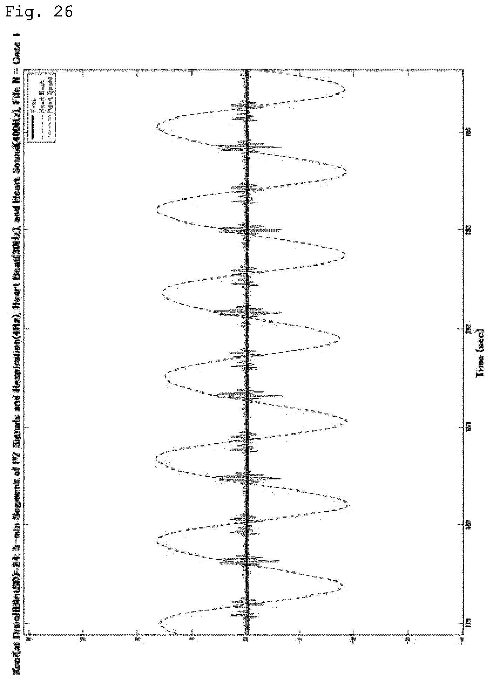

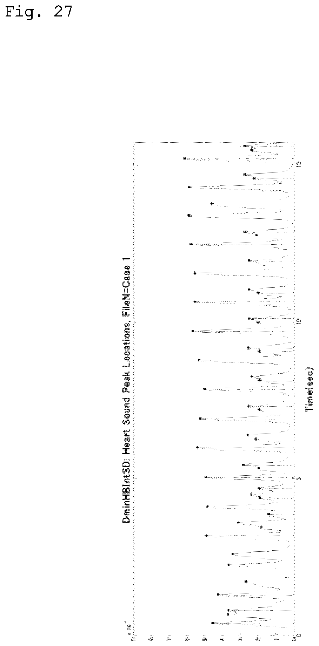

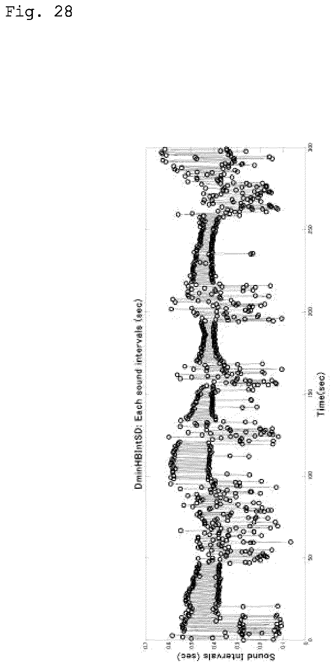

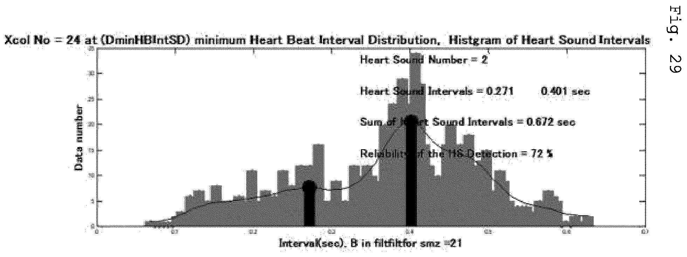

Fig. 26 shows the waveforms of the heart sound signal (black), heartbeat signal (red), and respiratory signal (blue) extracted from time window (7). Fig. 27 shows the amplitude variation waveform of the heart sound signal shown in Fig. 26. Fig. 28 shows distribution information indicating the distribution of peak time intervals detected from the amplitude variation waveform shown in Fig. 27, and Fig. 29 shows the distribution information in a histogram format.
In Figs. 28 and 29, groups of peak time intervals are not clear; however, as a result of automatically analyzing the histogram, it was determined that they were divided into 2 groups (i.e., it was predicted that heart sound signals did not include extra heart sounds). The average time interval of each group was 0.271 sec and 0.401 sec, and their total value (predicted heartbeat cycle) was 0.672 sec, whereas the average RR interval was 0.931 sec. Therefore, the prediction reliability was 0.672/0.931 = 72%.
From the above, the prediction based on the heart sound signals extracted from time windows (1), (5), and (6) included extra heart sounds, whereas the prediction based on the heart sound signal extracted from time window (5) did not include extra heart sounds. However, the reliability of the prediction based on the heart sound signals extracted from time windows (1), (3), and (4) was much higher than that of time window (5); thus, it can be predicted that the heart sounds of subject X include extra heart sounds.
Further, when the type of extra heart sound was analyzed by the extra heart sound identification unit 33f, the extra heart sound was identified as sound III.

Example 2

In Example 2, the target was subject Y, who had been diagnosed for the presence of sound III in the heart sounds by routine examination. Heart sound signals were extracted from the signals of the following time windows (5) and (6) in the piezoelectric signal obtained while subject Y was lying on the sheet sensor, and extra heart sounds were determined based on peak time intervals detected from the amplitude variation waveform of the heart sound signals.
(5) a time window of apnea in time window (2) above
(6) a time window in which the SD of the heartbeat amplitude is minimum

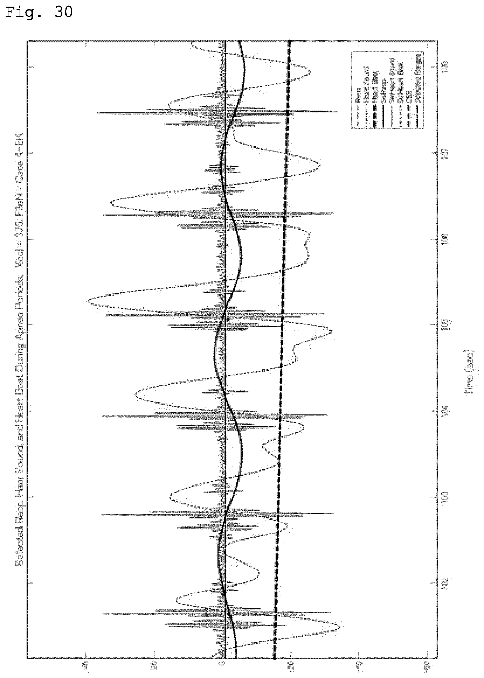

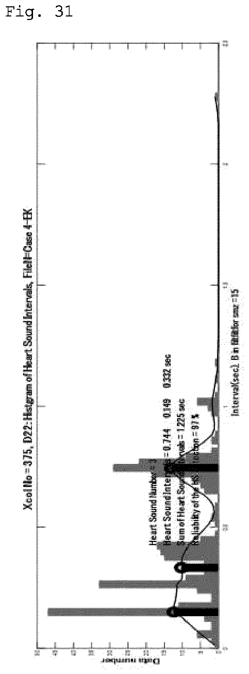

Fig. 30 shows the waveforms of the heart sound signal (black), heartbeat signal (red), CSR signal (pink), and respiratory signal (blue) extracted from time window (5). Fig. 31 shows the histogram of the distribution of peak time intervals detected from the amplitude variation waveform of the heart sound signal shown in Fig. 30.
Since the peak time intervals are roughly divided into 3 groups in Fig. 31, it can be predicted that the heart sound signals include extra heart sounds. The average time interval of each group was 0.149 sec, 0.332 sec, and 0.744 sec, and their total value (predicted heartbeat cycle) was 1.225 sec, whereas the average RR interval was 1.185 sec. Therefore, the prediction reliability was 1.185/1.225 = 97%.

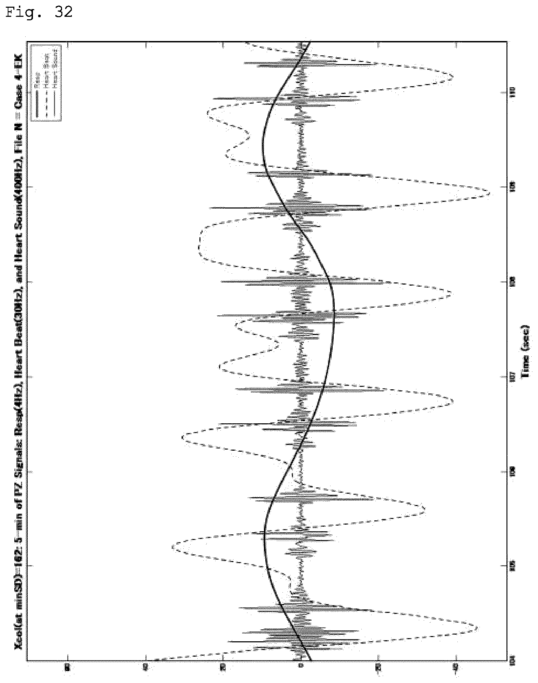

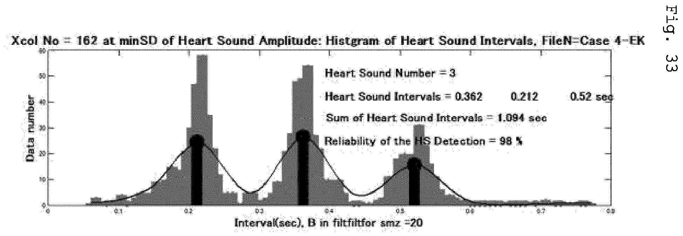

Fig. 32 shows the waveforms of the heart sound signal (black), heartbeat signal (red), and respiratory signal (blue) extracted from time window (6). Fig. 33 shows the histogram of the distribution of peak time intervals detected from the amplitude variation waveform of the heart sound signal shown in Fig. 32.
Since the peak time intervals are roughly divided into 3 groups in Fig. 33, it can be predicted that the heart sound signals include extra heart sounds. The average time interval of each group was 0.212 sec, 0.362 sec, and 0.52 sec, and their total value (predicted heartbeat cycle) was 1.094 sec, whereas the average RR interval was 1.116 sec. Therefore, the prediction reliability was 1.094/1.116 = 98%.
From the above, the prediction based on the heart sound signals extracted from time windows (5) and (6) included extra heart sounds, and the prediction reliability was high in both cases. Accordingly, it can be predicted that the heart sounds of subject Y include extra heart sounds.
Further, when the type of extra heart sound was analyzed by the extra heart sound identification unit 33f, the extra heart sound was identified as sound IV.

Reference Signs List

1. Monitoring system
2. Measuring device
21. Sheet sensor
22. Measurement unit
3. Management device (heart sound extraction device, heart sound analysis device)
30. Auxiliary storage device
31. Acquisition unit
32. Heart sound extraction unit
32a. Respiratory signal extraction unit
32b. Heartbeat signal extraction unit
32c. Heart sound signal extraction unit
32d. RST analysis unit
32e. CSR analysis unit
32f. Heartbeat analysis unit
32g. Time window setting unit
32h. Heart sound signal selection unit
33. Heart sound analysis unit
33a. Amplitude variation waveform generation unit
33b. Peak detection unit
33c. Time interval calculation unit
33d. Prediction support unit

33e. Distribution information generation unit
33f. Extra heart sound identification unit
34. Display unit
4. Smartphone
D1. Heart sound extraction program
D2. Heart sound analysis program

**Claims**

1. A heart sound extraction device comprising:

   an acquisition unit that, while a subject is lying on a sheet sensor that outputs a piezoelectric signal depending on the vibration applied, continuously acquires the piezoelectric signal output from the sheet sensor;
   a heart sound signal extraction unit that extracts the heart sound signal of the subject from the piezoelectric signal; and
   a time window setting unit that sets at least any of the following time windows in the extracted heart sound signal:

   (1) a time window in which the reciprocal of the standard deviation of respiratory frequency is maximum or equal to or greater than a predetermined value,
   (2) a time window in which the periodic respiratory power is maximum or equal to or greater than a predetermined value,
   (3) a time window in which the amplitude of the heart sound signal is maximum or equal to or greater than a predetermined value,
   (4) a time window in which the amplitude variation of the heart sound signal is minimum or equal to or less than a predetermined value,
   (5) a time window including the time zone of apnea in time window (2),
   (6) a time window in which the standard deviation of the heartbeat amplitude is minimum or equal to or less than a predetermined value, and
   (7) a time window in which the standard deviation of heartbeat intervals is minimum or equal to or less than a predetermined value.

2. The heart sound extraction device according to claim 1, wherein the time window setting unit sets a signal of a time window in which the reciprocal of the standard deviation of respiratory frequency is maximum from the piezoelectric signal.

3. The heart sound extraction device according to claim 1, wherein the time window setting unit sets a time window including the time zone of apnea in a time window in which the periodic respiratory power is maximum from the piezoelectric signal.

4. The heart sound extraction device according to claim 1, wherein the time window setting unit sets a time window in which the standard deviation of the heartbeat amplitude is minimum from the piezoelectric signal.

5. The heart sound extraction device according to any one of claims 1 to 4, further comprising a display unit that displays a waveform within the set time window of the extracted heart sound signal.

6. A heart sound extraction program for making a computer work as each unit of the heart sound extraction device according to any one of claims 1 to 5.

7. A computer-readable recoding medium that records the heart sound extraction program according to claim 6.

8. A monitoring system that monitors the state of a subject based on the heart sound of the subject, comprising:

   a sheet sensor that outputs a piezoelectric signal depending on the vibration applied; and
   a heart sound extraction device that extracts a heart sound signal from the piezoelectric signal output from the sheet sensor while the subject is lying on the sheet sensor,
   the heart sound extraction device being the heart sound extraction device according to any one of claims 1 to 5.

Fig. 1

Fig. 2

Fig. 3

Fig. 4

Fig. 5

(a)

Max RST Period Data

Respiratory cycle (respiratory rate) = 0.2 Hz (12/min)

RST = 300

CSR(0,1,2)grade = 0

(b)

Ventilatory, Noise, and CSR Signal Components

Fig. 6

(a) Max CSR period Data

Respiratory cycle (respiratory rate) = 0.257 Hz (15/min)

RST = 12

CSR(0,1,2)grade = 2

Frequency (Hz), Sampling Hz= 4Hz

(b) Ventilatory, Noise, and CSR Signal Components

Org Vent4
Noise Comp
Vent Comp
CSR Comp

Time (sec)

Fig. 7

Fig. 8

Fig. 9

Fig. 10

Fig. 11

Fig. 12

Fig. 13

Fig. 14

Fig. 15

Fig. 16

Fig. 17

D1(Max RSI periods): Heart Sound Peak Locations

Fig. 18

Fig. 19

Xcol No = 448 at Max RSI: Histgram of Heart Sound Intervals,FileN=Case 1

Heart Sound Number = 3

Heart Sound Intervals = 0.193    0.327    0.64 sec

Sum of Heart Sound Intervals = 1.16 sec

Reliability of the HS Detection = 96 %

Interval(sec), B in filtfilt for smz =21

Data number

Fig. 20

Selected Resp, Heart Sound, and Heart Beat During Apnea Periods, Xcol = 45, FileN = Case 1

Fig. 21

Fig. 22

Fig. 23

Fig. 24

Fig. 25

Xcol No = 155 at (DminHBampSD) minimum Heart Beat Amp Distribution, Histgram of Heart Sound Intervals

Heart Sound Number = 3

Heart Sound Intervals = 0.205    0.356    0.429 sec

Sum of Heart Sound Intervals = 0.99 sec

Reliability of HS Detection = 98 %

Data number

Interval(sec), B in filtfitfor smz =21

Fig. 26

Fig. 27

Fig. 28

Fig. 29

Fig. 30

Fig. 31

Fig. 32

Fig. 33

Xcol No = 162 at minSD of Heart Sound Amplitude: Histgram of Heart Sound Intervals, FileN=Case 4-EK

Heart Sound Number = 3

Heart Sound Intervals = 0.362       0.212       0.52 sec

Sum of Heart Sound Intervals = 1.094 sec

Reliability of the HS Detection = 98 %

Interval(sec), B in filtfiltfor smz =20

## INTERNATIONAL SEARCH REPORT

International application No.

**PCT/JP2022/043269**

### A. CLASSIFICATION OF SUBJECT MATTER

*A61B 5/02*(2006.01)i; *A61B 5/0245*(2006.01)i; *A61B 5/11*(2006.01)i; *A61B 5/113*(2006.01)i
FI:  A61B5/02 350; A61B5/11 100; A61B5/0245; A61B5/113

According to International Patent Classification (IPC) or to both national classification and IPC

### B. FIELDS SEARCHED

Minimum documentation searched (classification system followed by classification symbols)

A61B5/02; A61B5/11-5/113; A61B7/04

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII)

### C. DOCUMENTS CONSIDERED TO BE RELEVANT

| Category* | Citation of document, with indication, where appropriate, of the relevant passages | Relevant to claim No. |
|---|---|---|
| X | JP 2017-500998 A (SONOMEDICAL PTY LTD) 12 January 2017 (2017-01-12) paragraphs [0010], [0020], [0024]-[0025], [0064] | 1, 5-8 |
| Y | | 1-2, 4-8 |
| Y | JP 2020-75136 A (HEALTH SENSING CO LTD) 21 May 2020 (2020-05-21) paragraphs [0015], [0022], [0027], [0037] | 1, 4-8 |
| Y | US 2020/0077913 A1 (CARDIAC PACEMAKERS, INC.) 12 March 2020 (2020-03-12) paragraphs [0046], [0069], [0082] | 1-2, 5-8 |
| Y | WO 2021/111680 A1 (SUMITOMO RIKO CO LTD) 10 June 2021 (2021-06-10) paragraphs [0025], [0051] | 1-2, 5-8 |
| A | JP 2018-102736 A (AMI CO LTD) 05 July 2018 (2018-07-05) paragraph [0023] | 3 |
| A | WO 2011/019091 A1 (ASANOI, Hidetsugu) 17 February 2011 (2011-02-17) p. 28, line 21 to p. 30, line 22 | 3 |

☐ Further documents are listed in the continuation of Box C.  ☑ See patent family annex.

| | |
|---|---|
| *  Special categories of cited documents:<br>"A"  document defining the general state of the art which is not considered to be of particular relevance<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| Date of the actual completion of the international search | Date of mailing of the international search report |
|---|---|
| **13 December 2022** | **14 February 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2022/043269**

| Patent document cited in search report | | | Publication date (day/month/year) | Patent family member(s) | | | Publication date (day/month/year) |
|---|---|---|---|---|---|---|---|
| JP | 2017-500998 | A | 12 January 2017 | US | 2017/0035303 | A1 | |
| | | | | paragraphs [0022], [0032], [0036]-[0037], [0135] | | | |
| | | | | WO | 2015/089591 | A1 | |
| | | | | EP | 3082596 | A1 | |
| JP | 2020-75136 | A | 21 May 2020 | (Family: none) | | | |
| US | 2020/0077913 | A1 | 12 March 2020 | WO | 2020/050918 | A1 | |
| | | | | EP | 3846695 | A1 | |
| | | | | CN | 113164155 | A | |
| WO | 2021/111680 | A1 | 10 June 2021 | US | 2022/0142507 | A1 | |
| | | | | paragraphs [0036], [0062] | | | |
| | | | | EP | 3998020 | A1 | |
| | | | | CN | 114302676 | A | |
| JP | 2018-102736 | A | 05 July 2018 | US | 2019/0313943 | A1 | |
| | | | | paragraph [0023] | | | |
| | | | | WO | 2018/124173 | A1 | |
| WO | 2011/019091 | A1 | 17 February 2011 | US | 2012/0125337 | A1 | |
| | | | | paragraphs [0188]-[0200] | | | |
| | | | | EP | 2465434 | A1 | |
| | | | | CN | 102481127 | A | |
| | | | | KR | 10-2012-0062750 | A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **TAKASHI MURO** ; **MAY 2013**. Auscultation - Sounds III and IV - Tracking auscultatory findings to understand pathological changes. Medical Tribune